# EUROPÄISCHE PATENTSCHRIFT

(11) **EP 1 572 181 B1**
(45) Veröffentlichungstag und Bekanntmachung des Hinweises auf die Patenterteilung: **25.06.2008**
(21) Anmeldenummer: 03778298.4
(22) Anmeldetag: 03.11.2003
(51) Int. Cl.: A61K 31/195, A61K 31/216, A61K 31/381, A61P 13/10

(54) **PHARMAZEUTISCHE ZUSAMMENSETZUNG AUS EINEM BETA-3-ADRENOZEPTOR-AGONISTEN UND EINEM SEROTONIN- UND/ODER NOREPINEPHRIN-REUPTAKE-INHIBITOR UND DEREN VERWENDUNG ZUR BEHANDLUNG VON BLASENFUNKTIONSSTOERUNGEN**
PHARMACEUTICAL COMPOSITION COMPRISING A BETA-3-ADRENOCEPTOR AGONIST AND A SEROTONIN AND/OR NOREPINEPHRINE REUPTAKE INHIBITOR AND THE USE OF SAID COMPOSITION FOR TREATING BLADDER DYSFUNCTION
COMPOSITION PHARMACEUTIQUE CONTENANT UN AGONISTE BETA-3-ADRENORECEPTEUR ET UN INHIBITEUR DE RECAPTAGE DE LA SEROTONINE ET/OU DE LA NOREPINEPHRINE AINSI QUE L'UTILISATION DE CETTE COMPOSITION POUR LE TRAITEMENT DU DYSFONCTIONNEMENT DE LA VESSIE

(30) Priorität: 27.11.2002 EP 02026546
(43) Veröffentlichungstag der Anmeldung: 14.09.2005
(60) Teilanmeldung: 05027827.4
(73) Patentinhaber: Boehringer Ingelheim International GmbH, 55216 Ingelheim am Rhein (DE)
(72) Erfinder: MEHLBURGER, Ludwig, 55411 BINGEN (DE); MICHEL, Martin, NL-1071 ED AMSTERDAM (NL); WIENRICH, Marion, 64331 WEITERSTADT (DE); EBINGER, Ursula, Boonton Township, NJ 07005 (US)
(86) Internationale Anmeldenummer: PCT/EP2003/012225
(87) Internationale Veröffentlichungsnummer: WO 2004/047830

(56) Entgegenhaltungen:
- EP-A- 0 654 264
- EP-A- 0 958 835
- EP-A- 1 043 308
- EP-A- 1 072 583
- EP-A- 1 095 932
- EP-A- 1 258 253
- WO-A-01/62236
- WO-A-97/33880
- WO-A-02/053140
- WO-A-02/085871
- US-A- 5 441 985
- US-A1- 2002 010 216
- US-A1- 2002 128 247
- THOR K B ET AL: "EFFECTS OF DULOXETINE, A COMBINED SEROTONIN AND NOREPINEPHRINE REUPTAKE INHIBITOR, ON CENTRAL NEURON CONTROL OF LOWER URINARY TRACT FUNCTION IN THE CHLORALOSE-ANESTHETIZED FEMAL CAT" JOURNAL OF PHARMACOLOGY AND EXPERIMENTAL THERAPEUTICS, AMERICAN SOCIETY FOR PHARMACOLOGY AND, US, Bd. 274, Nr. 2, 1995, Seiten 1014-1024, XP009023623 ISSN: 0022-3565
- SHARMA A ET AL: "PHARMACOKINETICS AND SAFETY OF DULOXETINE, A DUAL-SEROTONIN AND NOREPINEPHRINE REUPTAKE INHIBITOR" JOURNAL OF CLINICAL PHARMACOLOGY, LIPPINCOTT CO, HAGERSTOWN, MD, US, Bd. 40, Nr. 2, Februar 2000 (2000-02), Seiten 161-167, XP000904872 ISSN: 0091-2700
- SALETU B ET AL: "TANDAMINE - A NEW NOREPINEPHRINE REUPTAKE INHIBITOR CLINICAL, PSYCHOMETRIC AND QUANTITATIVE EEG STUDIES IN DEPRESSED PATIENTS" INTERNATIONAL PHARMACOPSYCHIATRY, KARGER, BASEL, CH, Bd. 12, Nr. 3, 1977, Seiten 137-152, XP000605654 ISSN: 0020-8272
- YAMANISHI TOMONORI ET AL: "The role of beta(3)-adrenoceptors in mediating relaxation of porcine detrusor muscle." BRITISH JOURNAL OF PHARMACOLOGY. ENGLAND JAN 2002, Bd. 135, Nr. 1, Januar 2002 (2002-01), Seiten 129-134, XP002272188 ISSN: 0007-1188
- ANDERSSON K-E: "THE OVERACTIVE BLADDER: PHARMACOLOGIC BASIS OF DRUG TREATMENT" UROLOGY, BELLE MEAD, NJ, US, Bd. 50, Nr. 6, SUPPL 1, 1997, Seiten 74-84, XP001189072 ISSN: 0090-4295
- SELLERS DONNA J ET AL: "Potential therapeutic targets for treatment of the overactive bladder" WORLD JOURNAL OF UROLOGY, SPRINGER INTERNATIONAL, DE, Bd. 19, Nr. 5, 2001, Seiten 307-311, XP002971377 ISSN: 0724-4983
- SPRINGER J P ET AL: "FACILITATORY AND INHIBITORY EFFECTS OF SELECTIVE NOREPINEPHRINE REUPTAKE INHIBITORS ON HYPOGASTRIC NERVE-EVOKED URETHRAL CONTRACTIONS IN THE CAT: A PROMINENT ROLE OF URETHRAL BETA-ADRENERGIC RECEPTORS" JOURNAL OF UROLOGY, BALTIMORE, MD, US, Bd. 152, Nr. 2, PART 1, August 1994 (1994-08), Seiten 515-519, XP008020464 ISSN: 0022-5347

## Beschreibung

Diese Erfindung beschreibt eine neue Wirkstoffkombination zur Behandlung von Harnblasenfunktionsstörungen, insbesondere der Mischinkontinenz. Erfindungsgemäß wird eine pharmazeutische Wirkstoffkombination aus wenigstens einem beta-3-Adrenozeptor-Agonisten und wenigstens einem Serotonin- und/oder Norepinephrin-Reuptake-Inhibitor gemäß Anspruch 1 vorgestellt.

### Stand der Technik

Die Inzidenz der Harninkontinenz nimmt durch die Verschiebung der Altersstruktur immer mehr zu. Dennoch werden die Betroffenen zum großen Teil immer noch nicht oder nicht adäquat behandelt. Neben den medizinischen Folgeerkrankungen, wie chronische Harnweginfektionen, ist Harninkontinenz für die Betroffenen mit einem hohen psychischen Leidensdruck verbunden. Schätzungsweise sind 100 Millionen älterer Menschen von Harninkontinenz betroffen.

Der untere Harntrakt besteht aus der Harnblase, der Harnröhre (Urethra), den entsprechenden Muskeln und der Ligamente des Halteapparates. Die Aufgabe der Harnblase besteht in der Speicherung des Harns und dessen Entleerung. Für die Erfüllung der Speicherfunktion ist nicht nur die Relaxation des Harnblasenmuskels (Detrusormuskel), sondern auch Verschlussmechanismen durch den Blasenhals, die glatte Muskulatur der Urethra sowie die quergestreifte Muskulatur der Urethra und des Beckenbodens von Bedeutung. Bei der Harnblasenentleerung (Miktion) kontrahiert sich der Detrusormuskel, während sich Urethra und Beckenboden entspannen bzw. der Harnblasenschließmuskel sich öffnet. Diese Vorgänge bedürfen einer komplizierten Steuerung durch das parasympathische, sympathische und somatische Nervensystem.

Hamblasenfunktionsstörungen stellen eine heterogene Gruppe von Störungen dar, die sich bezügliche ihrer Ätiologie, der Diagnose und der Therapie unterscheiden.

In den Standardisierungsempfehlungen der International Continence Society (ICS) wird Harninkontinenz definiert als unwillkürlicher Harnverlust, der objektiv nachweisbar ist und ein soziales und hygienisches Problem darstellt. Im Allgemeinen tritt Harninkontinenz nur dann auf, wenn es während der Speicherphase unbeabsichtigt zu einem Anstieg des Druckes in der Blase kommt. Dies kann infolge von ungehemmten Kontraktionen des Detrusormuskels (Dranginkontinenz) oder Inkompetenz des urethralen Verschlussmechanismus (Stressinkontinenz) geschehen.

Gemäß der Definition der ICS spricht man von einer überaktiven Blase (Overactive Bladder; OAB) bei nicht unterdrückbaren, imperativen Harndrang, verbunden mit oder ohne Dranginkontinenz, gewöhnlich mit erhöhter Miktionsfrequenz und nächtlichem Wasserlassen. Pathophysiologisch können dieser Erkrankung unwillkürliche Kontraktionen während der Füllphase zugrunde liegen, deren Ursache neurogenener bzw. nicht-neurogener (idiopathischer) Natur sein können.

Dranginkontinenz ist gekennzeichnet durch unwiderstehlichen Harndrang und unwillkürlichem Urinverlust.

Stressinkontinenz ist durch den unfreiwilligen Urinverlust gekennzeichnet, der in der Regel bei Auftreten eines erhöhten intraabdominalen Drucks auftritt. Dies kann beispielsweise beim Heben, Husten, Niesen, Laufen und bei gleichzeitig fehlender Detrusoraktivität auftreten. Zu dem Harnverlust kommt es infolge einer variablen Kombination einer Insuffizienz der Harnblasenschließmuskulatur und Beckenbodens sowie eines anatomischen Defektes des Halteapparates. In der Folge wird der Verschlussdruck der Urethra zu niedrig und Inkontinenz ist die Folge. Die reine Stressinkontinenz tritt häufig bei Frauen aus, insbesondere wenn sie geboren haben. Bei Männern wird diese Form der Harninkontinenz meist nur nach Prostatektomien oder anderen chirurgischen Eingriffen des kleinen Becken beobachtet.

Bei der sog. Mischinkontinenz leiden Patienten sowohl an Symptomen der Stressinkontinenz wie auch der Dranginkontinenz. Auch hiervon sind wieder besonders Frauen betroffen.

Für die Therapie der verschiedenen Formen von Harnblasenfunktionsstörungen, insbesondere der Stressinkontinenz, Dranginkontinenz, Mischinkontinenz oder der hyperaktiven Blase stehen verschiedene Behandlungsansätze zur Verfügung.

Zur Therapie der Dranginkontinenz empfiehlt die WHO die Behandlung mit Anticholinergika (Antimuskarinika). Allerdings ist deren Einsatz aufgrund einer nur moderaten Wirksamkeit und vor allem der erheblichen Nebenwirkungen wie Mundtrockenheit, Akkomodationsstörungen, Obstipation, zentralnervösen Wirkungen (Schwindel, Müdigkeit, Verwirrtheit) limitiert.

Für die Behandlung der Stressinkontinenz stehen besonders konservative und chirurgische Maßnahmen zur Verfügung. Eine allgemein anwendbare medikamentöse Therapie konnte sich bisher nicht etablieren. Alpha-Agonisten, wie Pseudoephedrin und Phenylpropanolamin zeigen bei der Behandlung einer geringgradigen Stressinkontinenz eine, jedoch äußerst moderate Wirkung. Nachteilig ist, dass diese keine Selektivität für die Urethralmuskulatur besitzen und mit häufigen Nebenwirkungen wie Hypertonie, Tachykardie, Arrhythmie, Schlafstörungen, Kopfschmerzen und Tremor verbunden sind.

Die Therapie der Mischinkontinenz wird kontorvers diskutiert und umfasst Kombinationen von invasiven Verfahren zur Behandlung der Stressinkontinenzkomponente und medikamentöse Verfahren zur Behandlung der Dranginkontinenzkomponente.

Seit Mitte der 1995er Jahre wird davon berichtet, dass auch selektive beta-3-Adrenozeptor-Agonisten in der Therapie der Harninkontinenz erfolgsversprechend sind (EP 0 958 835). Da der Stimulation von beta-3-Rezeptoren für die Relaxation des Detrusormuskels eine außerordentliche Bedeutung zukommt, sollte der Einsatz von selektiven beta-3-Adrenozeptoren bei Patienten mit Dranginkontinenz in einer Reduktion bzw. einer Verhinderung von unwillkürlichen Detrusorkontraktionen während der Harnspeicherphase resultieren. Versuche mit beta-3-Adrenozeptoragonisten versprechen eine hohe Wirksamkeit bei guter Verträglichkeit. Daneben sollte deren Wirkung auf die Speicherphase der Harnblase beschränkt bleiben und eine ungestörte Blasenentleerung ohne Restharnbildung garantiert sein.

In der klinischen Prüfung zur Behandlung der Stressinkontinenz sind derzeit solche Substanzen, die selektiv Serotonin und/oder Norepinephrin Wiederaufnahme aus dem synaptischen Spalt in die Nervenzellen hemmen. Diese Substanzen werden selektive Serotonin-/Norepinephrin-Reuptake-Inhibitoren genannt und führen zu einer Verlängerung und Verstärkung der Wirkung von Serotonin (5-Hydroxytryptamin = 5-HT), und/oder Noradrenalin (NA). Über einen komplexen Wirkmechanismus vermögen selektive Serotonin-/Norepinephrin-Reuptake-Inhibitoren die Aktivität des N. Pudendus zu stimulieren, die Kontraktion des quergestreiften Harnblasenschließmuskels zu fördern und so den Kontinenzerhalt zu sichern. Klinische Studien der Phase III konnten inzwischen zeigen, dass unter Verwendung eines selektiven Serotonin-/Norepinephrin-Reuptake-Inhibitoren erstmals eine wirksame medikamentöse Therapie der Stressinkontinenz möglich ist.

Auch zur Therapie der hyperaktiven Blase stehen nur beschränkte Therapiemöglichkeiten zur Verfügung. Zu den wenigen etablierten Behandlungsformen gehören auch hier Medikamente mit Antimuskarinika als aktivem Wirkstoff.

### Aufgabe der Erfindung

Trotz der viel versprechenden Ansätze und Fortschritte zur Behandlung der verschiedenen Formen der Harninkontinenz, die sich kausal komplex und heterogen darstellen, bleibt die Entwicklung effizienter und verträglicher Therapien eine Herausforderung.
Mit der vorliegenden Erfindung soll ein solcher Beitrag zur Therapie der Harninkontinenz im Allgemeinen, sowie den verschiedenen vorstehend genannten Ausbildungsformen, geschaffen werden. Dafür wird eine pharmazeutische Zusammensetzung gemäß Anspruch 1 vorgestellt, die sowohl die Vorteile der Serotonin- und/oder Norepinephrin-Reuptake-Inhibitoren als auch diejenigen der beta-3-Adrenozeptor-Agonisten in einer die Therapie der Grunderkrankung begünstigender Art und Weise miteinander verbinden soll.

### Beschreibung der Erfindung

Gemäß der vorliegenden Erfindung wird eine neue pharmazeutische Zusammensetzung bereitgestellt, die (a) wenigstens einen Serotonin- und/oder Norepinephrin-Reuptake-Inhibitor in einer pharmazeutisch wirksame Menge und (b) einen beta-3-Adrenozeptor-Agonisten gemäß Anspruch 1 in einer pharmazeutisch wirksame Menge als aktive Bestandteile aufweist.

### a) aktive Komponenten

Bei der Beschreibung der bevorzugten Ausführungsform soll im weiteren aus Gründen der Klarheit eine gewisse Terminologie verwendet werden. Eine derartige Terminologie soll die angeführte Ausführungsform sowie alle technischen Äquivalente umfassen, die auf ähnliche Weise für einen ähnlichen Zweck zur Erzielung eines ähnlichen Ergebnisses wirken. Eingeschlossen sind pharmakologisch annehmbare Salze derselben. Beispiele für pharmazeutisch wirksame Salze für jede der Verbindungen, die Gegenstand dieser Beschreibung sind, schließen Salze ein, die aus pharmazeutisch annehmbaren Säuren oder Basen, einschließlich organischer und anorganischer Säuren und Basen, hergestellt sind. Wenn die zur Verwendung bevorzugte Verbindung basisch ist, können Salze aus pharmazeutisch annehmbaren Säuren hergestellt werden. Bei der Auswahl des bevorzugtesten Salzes, bzw. zur Klärung ob ein Salz oder die Neutralverbindung eingesetzt wird, werden u.a. Eigenschaften wie Bioverfügbarkeit, Herstellbarkeit, Verarbeitbarkeit und Lagerfähigkeit berücksichtigt. Geeignete pharmazeutisch annehmbare Säuren umfassen Essig-, Benzolsulfon- (Besylat-), Benzoe-, p-Bromphenylsulfon-, Camphersulfon-, Kohlen-, Citronen-, Ethansulfon-, Fumar-, Glucon-, Glutamin-, Bromwasserstoff , Chlorwasser-, Jodwasserstoff-, Isethion-, Milch-, Malein-, Äpfel-, Mandel-, Methansulfon- (Mesylat-), Mucin-, Salpeter-, Oxal-, Pamoa-, Pantothen-, Phosphor-, Bernstein-, Schwefel-, Wein-, p-Toluolsulfonsäure und dergleichen. Beispiele für derartige pharmazeutisch annehmbare Salze umfassen Acetat, Benzoat, Hydroxybutyrat, Bisulfat, Bisulfit, Bromid, Butin-1,4-dioat, Caproat, Chlorid, Chlorbenzoat, Citrat, Dihydrogenphosphat, Dinitrobenzoat, Fumarat, Glycollat, Heptanoat, Hexin-1,6-dioat, Hydroxybenzoat, Iodid, Lactat, Maleat, Malonat, Mandelat, Metaphosphat, Methansulfonat, Methoxybenzoat, Methylbenzoat, Monohydrogenphosphat, Naphthalin-1-sulfonat, Naphthalin-2-sulfonat, Oxalat, Phenylbutyrat, Phenylproprionat, Phosphat, Phthalat, Phenylacetat, Propansulfonat, Propiolat, Propionat, Pyrophosphat, Pyrosulfat, Sebacat, Suberat, Succinat, Sulfat, Sulfit, Sulfonat, Tartrat, Xylolsulfonat und dergleichen.

In dem Ausmaß, wie es zur Vervollständigung erforderlich ist, werden die Synthese der Verbindungen, für die Stand der Technik angeführt wird, und deren Dosierungen ausdrücklich durch Bezugnahme auf den an der entsprechenden Stelle zitierten Stand der Technik aufgenommen.

Der selektive Serotonin- und/oder Norepinephrin-Reuptake-Inhibitor ist Duloxetin, N-Methyl-3-(1-naphthalinyloxy)-3-(2-thienyl)propanamin und dessen pharmazeutisch annehmbare Salze entweder in seiner enantiomeren (insbesondere der (+)-enantiomeren) oder racemischen Form und in den bevorzugtesten Fällen der (+)-Form.

In der englischen Literatur trägt die Verbindung ein e am Namensende, d.h Duloxetine statt Duloxetin.

Duloxetin, (+)-N-Methyl-3-(1-naphthalinyloxy)-3-(2-thienyl)propanamin, wird in den US-Patenten Nr. US 4,956,388 oder US 5,023,269 offenbart. Nach der US 5,744,474 kann die Verbindung verwendet werden, um Harninkontinenz zu behandeln. Duloxetine wird durch die folgende Formel I dargestellt: 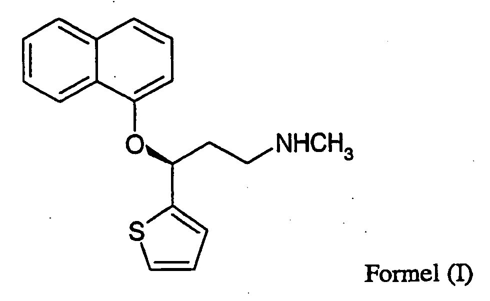 Duloxetin wird bevorzugt in Form des Hydrochlorid-Salzes und als (+)-Enantiomer verwendet.

Duloxetin kann in der Indikation Harninkontinenz, inklusive der eingangs gelisteten Subindikationen Stressinkontinenz, Dranginkontinenz, Mischinkontinenz oder hyperaktive Blase, verwendet werden.

Die zweite Komponente umfasst einen oder mehrere beta-3-Adrenorezeptor-Agonisten, augsgewälhlt aus der Gruppe bestehend aus (-)-Ethyl-2-[4-(2-{[(1S,2R)-2-hydroxy-2-(4-hydroxyphenyl)-1-methylethyl]amino}ethyl)-2,5-dimethylphenyloxy]acetat oder (-)-2-[4-(2-{[(1S,2R)-2-hydroxy-2-(4-hydroxyphenyl)-1-methylethyl]-amino}ethyl)-2,5-dimethylphenyloxy]essigsäure und pharmakologisch aktive Salze derselben.

Diese Verbindungen sind in der WO 00/02846 oder der WO 2003024916 offenbart.

Diese zwei namentlich genannten Verbindungen werden durch die folgende Formel II dargestellt, die im Fall von Widersprüchlichkeiten gegenüber dem vorstehenden Namen vorherrschen soll: 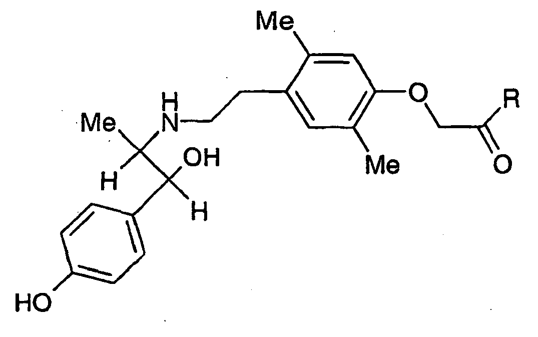 bei R = OEthyl: (-)-Ethyl-2-[4-(2-{[(1S,2R)-2-hydroxy-2-(4-hydroxyphenyl)-1-methylethyl]amino}ethyl)-2,5-dimethylphenyloxy]acetat, vorzugsweise das Monohydrat, bei R = OH: (-)-2-[4-(2-{[(1S,2R)-2-Hydroxy-2-(4-hydroxyphenyl)-1-methylethyl]-amino}ethyl)-2,5-dimethylphenyloxy]essigsäure.

### b) Dosierung

Um die optimale Dosis der beiden Wirkstoffe für die Harninkontinenz zu bestimmen, müssen verschiedene Rahmenbedingungen berücksichtigt werden, wie beispielsweise Alter und Körpergewicht des Patienten, Natur und Stadium der Erkrankung, sowie die Potenz der Verbindung. Dies wird als im Vermögen des Fachmanns liegend angesehen, und man kann die bestehende Literatur über die Komponenten zu Rate ziehen, um die optimale Dosierung zu bestimmen. Die angegebenen Dosierungen beziehen sich auf die Dosierung nach Beendigung der Einstellungsphase.

Die im Folgenden angegebenen Dosierungen schließen ausdrücklich alle numerischen Werte, ganze oder gebrochene, innerhalb des angeführten Bereichs ein. Die Angaben beziehen sich auf erwachsene Menschen. Pädiatrische Dosierungen können geringer sein.

Die für den Menschen bevorzugte Dosis des Serotonin und/oder Norepinephrin-Reuptake-Inhibitors liegt zwischen 0,001 mg und 5 g pro Tag, bevorzugt beträgt sie zwischen 0,001 mg und 250 mg und ganz besonders bevorzugt liegt sie zwischen 0,1mg und 200 mg.

In einigen Fällen kann auch eine geringere Menge genügen, während in anderen Fällen eine größere Gesamtmenge notwendig sein kann.

Die tägliche Gesamtdosis kann in Abhängigkeit des Therapieregiments an einem Stück oder innerhalb von mehreren Portionen eingenommen werden. Das Therapieregiment kann auch Abstände zwischen den Einnahmen vorschreiben, die länger als ein Tag sind.

Die Auswahl der Dosierung der ersten Komponente, d.h. des Serotonin- und/oder Norepinephrin Reuptake Inhibitors, ist diejenige, die für eine Erleichterung des Patienten sorgen kann.

Wünschenswerterweise enthält, wenn Duloxetin als Wirkstoff gewählt wird, die tägliche Dosis etwa 0,1 mg bis etwa 500 mg. Bevorzugter enthält jede Dosis der Komponente etwa 0,5 bis etwa 160 mg des Wirkstoffs. Diese Dosierungsform gestattet, dass die volle tägliche Dosis in halben oder ganzen, einmaligen oder mehrmaligen Dosen verabreicht wird. Mehr als einmal tägliche oder zweimal tägliche Verabreichungen (z.B. 3, 4, 5 oder 6 Verabreichungen pro Tag) werden ebenfalls ausdrücklich hierin in Betracht gezogen.

Wie es wohlbekannt ist, hängen die Dosierungen und das Verabreichungsschema (d.h. eine, zwei, drei oder mehr Verabreichungen pro Tag) der zweiten Komponente von den Faktoren ab, auf die in Verbindung mit der Dosierungswahl der ersten Komponente Bezug genommen wurde.

Die durchschnittliche tägliche Erwachsenendosis der zweiten Komponente (beta-3-Agonist) beträgt etwa 10 mg bis etwa 750 mg pro Tag, bevorzugt 5 bis 120 mg, stärker bevorzugt 10 bis 100 mg, verabreicht in einer oder mehreren Dosen. Die Dosierungen schließen ausdrücklich alle numerischen Werte, ganze oder gebrochene, innerhalb des angeführten Bereichs ein.

Für beide Komponenten können die pädiatrischen Dosierungen geringer sein.

### c) Applikationsformen

Die Zusammensetzungen der vorliegenden Erfindung können zweckmäßigerweise in einer pharmazeutischen Zusammensetzung verabreicht werden, welche die aktiven Komponenten in Kombination mit einem geeigneten Träger enthält. Derartige pharmazeutische Zusammensetzungen können durch Verfahren hergestellt werden und Träger enthalten, die in der Technik wohlbekannt sind. Dem Fachmann stehen diesbezüglich allgemein anerkannte Fachwerke zur Verfügung.

Die Zusammensetzungen der vorliegenden Erfindung können parenteral (z.B. durch intravenöse, intraperitoneale, subkutane oder intramuskuläre Injektion), topisch, oral, intranasal, intravaginal, transdermal, rektal, pulmonal inhalativ oder nasal inhalativ verabreicht werden, wobei die orale Verabreichung besonders bevorzugt ist. Unter den oralen Verabreichungsformen sind magensaftresistente Formulierungen bevorzugt. Daher sind magensaftresistente Kapseln oder magensaftresistente Tabletten bevorzugt, was in beiden Fällen z.B. mit einem magensaftresistenten Überzug realisiert werden kann. Es wird besonders darauf geachtet, dass der Serotonin- / Norepinehrin-Reuptake Inhibitor in einer magensaftresistenten Darreichungsform formuliert wird. Der Fachmann findet für magensaftresistente Formulierungen im Stand der Technik Anleitungen.

Im folgen werde verschiedene Formulierungsoptionen gegeben. Der Fachmann kann hieraus eine geeignete Formulierung heraussuchen.

Für die orale therapeutische Verabreichung kann die erfindungsgemäße Zusammensetzung mit einem oder mehreren Trägem vereinigt werden und in Form von einnehmbaren Tabletten, bukkalen Tabletten, Sublingualtabletten, zuckerüberzogenen Tablette, Pulvern, Pudern, Pastillen, Dragees, Granulaten, Kapseln, Elixieren, Suspensionen, Lösungen, Sirupen, Oblaten, Kaugummis, Nahrungsmitteln und dergleichen verwendet werden.

Ein Pulver kann beispielsweise hergestellt werden, in dem die Partikel der aktiven Substanz durch Mahlen auf eine geeignete Größe gebracht werden.
Verdünnte Pulver können dadurch hergestellt werden, dass die pulverförmige Substanz mit einem untoxischen Trägermaterial, wie beispielsweise Laktose fein vermahlen und als Pulver ausgebracht wird. Andere diesbezüglich geeignete Trägermaterialien sind andere Kohlenhydrate, wie Stärke oder Mannitol. Gegebenenfalls können diese Pulver Geschmacksstoffe, Konservierungsstoffe, Dispergierungsagentien, Farbmittel und andere pharmakologische Hilfsstoffe enthalten.

Kapseln können ausgehend von einem Pulver der oben genannten Art oder anderen Pulvern hergestellt werden, die in eine Kapsel, bevorzugt eine Gelatinekapsel, eingebracht werden und die Kapsel danach geschlossen wird.

Es ist auch möglich, dass aus dem Stand der Technik bekannte Schmierstoffe in die Kapsel eingebracht werden oder für den Verschluss der beiden Kapselteile verwendet werden. Die Wirksamkeit einer Kapsel bei oraler Einnahme kann dadurch verstärkt werden, dass disintegrierende oder solubilisierende Stoffe hinzugegeben werden, wie beispielsweise Carboxymethylzellulose, Carboxymethylzellulosecalcium, niedrig substituierte Hydroxyprophylzellulose, Calciumcarbonat, Natriumcarbonat und andere Stoffe. Der Wirkstoff kann in der Kapsel nicht nur als Feststoff, sondern auch suspendiert vorliegen, beispielsweise in Pflanzenöl, Polyethylenglykol, Glycerol mit Hilfe von oberflächenaktiven Substanzen usw.

Tabletten können hergestellt werden, indem die pulverförmige Mischung gepresst wird und anschließend z.B. zu Granulaten weiterverarbeitet wird. Die Tabletten können verschiedene Hilfsstoffe beinhalten, wie z.B. Stärken, Milchzucker, Rohrzucker, Glukose (z.B. für Vaginaltabletten), Natriumchlorid, Harnstoff für Lösungs- u. Injektionstabletten, Amylose, verschieden Zellulosearten wie oben beschrieben und andere.
Als Feuchthaltemitte können beispielsweise Glycerin oder Stärke verwendet werden.

Als Sprengmittel können beispielsweise Stärke, Alginsäure, Calciumalginat, Pektinsäure, pulverisierter Agar-Agar, Formaldehydgelatine, Calciumcarbonat, Natriumbicarbonat, Magnesiumperoxid, Amylose verwendet werden.

Als Gegensprengmittel oder Lösungsverzögerer kommen beispielsweise Rohrzucker, Stearin, festes Paraffin, (bevorzugt mit einem Schmelzbereich von 50-52°C); Kakaofett, hydrierte Fette in Betracht.

Weitere Zerfallsmittel können sein: Maisstärke, Kartoffelstärke, Algininsäure und dergleichen.

Als Resorptionsbeschleuniger eignen sich unter anderem quaternäre Ammoniumverbindungen, Natriumlaurylsulfat, Saponine.

Als Bindemittelverteiler kann z.B. Ether verwendet werden und als Hydrophilisierungsmittel beziehungsweise als Zerfallsbeschleuniger Cetylalkohol, Glycerinmonostearat, Stärke, Maisstärke, Milchzucker, Netzmittel (z.B. Aerosol OT, Pluronics, Tweens), Tragantgummi, Gummi arabicum, Gelatine und andere.

Als Süßungsmittel können Saccharose, Fructose, Lactose oder Aspartam eingesetzt werden oder als Geschmacksmittel Pfefferminz, Wintergrünöl, Kirschgeschmack u.v.m.

Im Übrigen kommen als weitere Hilfsstoffe ganz allgemein in Betracht: Aerosil, Aerosol OT Ethylcellulose, Amberliteharz, XE-88, Amijel, Amisterol, Amylose, Avicel microcrystalline-cellulose, Bentonit, Calciumsulfat, Carbowax 4000 u. 6000, Carrageenan, Castorwax, Cellulose, Cellulose microcristalline, Crospovidone, Dextrane, Dextrin, Dicalciumphosphat, Grundmasse für pharmazeutische Tabletten, Kaolin, Laktose (USP), Lactosil, Magnesiumstearat, Mannit, Mannitol granular N. F. Methylcellulose, Miglyol 812 Neutralöl, Milchpulver, Milchzucker, nal-tab, Nepol-Amylose, Pöfizer crystalline sorbitol, Plasdone, Polyethylenglykole, Polyvinylacetatphthalat, Polyvinylpyrrolidon, Précirol, Rinderklauenöl (hydriert), Schmelztablettengrundmasse, Silicone, Stabiline, Starx 1500, Syloid, Tablettengrundmasse Waldhof, Tablettol, Talcum cetylatum u. stearatum, Tego-Metallseifen, Traubenzucker. u. Tylose. Besonders geeignet ist das Tablettierhilfsmittel K (M25), das im übrigen den Anforderungen der nachfolgenden Pharmakopoen entspricht: DAB, Ph, Eur, BP u. NF.

Weitere einsetzbare Hilfsstoffe finden sich in den Beispielen, aber auch andere Hilfsstoffe aus dem Stand der Technik können verwendet werden.

Die Tabletten können beispielsweise durch Direktverpressung hergestellt werden.

Auch andere oral applizierbare Formulierungen wie Lösungen, Sirup, Elixier usw. können hergestellt werden. Gegebenenfalls kann die Verbindung mikroverkapselt werden.

Eine parenterale Verabreichung kann dadurch erreicht werden, dass die Verbindung in einer Flüssigkeit gelöst wird und subkutan, intramuskulär oder intravenös injiziert wird. Als Lösungsmittel eignen sich beispielsweise Wasser oder ölige Medien.

Zur Herstellung von Suppositorien kann die Verbindung mit niedrigschmelzenden und wasserlöslichen oder wasserunlöslichen Materialien wie Polyethylenglykol, Kakaobutter, höheren Estern (beispielsweise Moerysthyl, Palmitat) oder Gemischen daraus formuliert werden.

Die obige Auflistung ist lediglich beispielhaft, und ein Fachmann könnte andere Hilfsstoffe in Betracht ziehen.

Verschiedene andere Materialien können als Überzüge vorhanden sein oder um auf andere Weise die physikalische Form der festen Einheitsdosierungsform zu modifizieren. Zum Beispiel können Tabletten, Pillen oder Kapseln mit Gelatine, Wachs, Schellack oder Zucker und dergleichen beschichtet sein. Wie bereits erwähnt sind für die oralen Darreichungsformen magensaftresistente Formulierungen bevorzugt. Daher sind magensaftresistente Überzüge für Tabletten oder Kapseln bevorzugt. Im Fall eines Sirup oder Elixiers kann Saccharose oder Fructose als Süßungsmittel, Methyl- und Propylparaben als Konservierungsmittel, einen Farbstoff und ein Geschmacksmittel, wie Kirsch- oder Orangengeschmack, enthalten sein.

Die genannten Hilfsstoffe sind dabei nicht auf die Verwendung der Applikationsform beschränkt, in deren Zusammenhang sie genannt worden sind, sondern können auch auf die anderen Applikationsformen übertragen werden.

Natürlich sollte jegliches Material, das bei der Herstellung von jeglicher Einheitsdosierungsform verwendet wird, pharmazeutisch annehmbar und in den verwendeten Mengen im wesentlichen nicht-toxisch sein. Zusätzlich können die aktiven Komponenten Präparaten mit verzögerter Freisetzung und Vorrichtungen einverleibt werden, welche, ohne darauf beschränkt zu sein, diejenigen einschließen, die auf osmotischen Drücken beruhen, um ein gewünschtes Freisetzungsprofil zu erzielen. Einmal-täglich-Formulierungen für jede der aktiven Komponenten sind speziell eingeschlossen.

Derartige Zusammensetzungen und Präparate sollten mindestens 0,1 % aktive Verbindung enthalten. Der Prozentsatz der Zusammensetzungen und Präparate kann natürlich variiert werden und kann zweckmäßig zwischen etwa 0,1 bis etwa 100 % des Gewichts einer gegebenen Einheitsdosierungsform ausmachen. Die Menge an aktiver Verbindung in derartigen therapeutisch nützlichen Zusammensetzungen ist derart, dass ein wirksame Dosierungsmenge erhalten wird.

Die erfindungsgemäße Zusammensetzung, welche die zwei aktiven Komponenten enthält, kann in derselben physikalischen Form oder gleichzeitig im Einklang mit den oben beschriebenen Dosierungen und in den oben beschriebenen Zufuhrvehikeln verabreicht werden. Die Dosierungen für jede aktive Komponente können getrennt abgemessen werden und können als einzige kombinierte Dosis verabreicht werden oder getrennt verabreicht werden. Sie können zur gleichen oder zu verschiedenen Zeiten verabreicht werden, solange beide aktiven Bestandteile zu einer Zeit über einen 24-stündigen Zeitraum in dem Patienten zur Wirkung kommen. Bevorzugt ist, wenn die beiden Komponenten so zur Wirkung kommen, dass eine Wirkung erzielt wird, die gegenüber der jeweiligen Einzelwirkung verbessert ist. Gleichzeitige oder zusammenfallende Verabreichung bedeutet, dass der Patient einen Arzneistoff innerhalb von etwa 5 Minuten nach Einnahme des anderen Arzneistoffes einnimmt. Aus Gründen der einfachen Handhabung sind Formulierungen bevorzugt, bei denen die beiden Arzneistoffe dem Patienten nahe beieinander und typisch gleichzeitig verabreicht werden. Danach könnte gegebenenfalls der Zeitpunkt der Verabreichung jedes Arzneistoffes nicht so wichtig sein.

### d) Indikationen

Die erfindungsgemäßen Arzneimittelzusammensetzung kann bevorzugt zur Behandlung oder Prophylaxe u.a. jedes der im Folgenden genannten Krankheitsbilder, als einzelnes Krankheitsbild wie auch in Kombination mit einem anderen der genannten Krankheitsbilder, eingesetzt werden, ohne jedoch darauf beschränkt zu sein.
Harninkontinez, insbesondere Stressinkontinezn, Dranginkontinez, Mischinkontinenz oder hyperaktive Blase neurogenen oder nicht-neurogenen Ursprungs und deren weiteren Subindikationen.

Erfindungsgemäß werden dabei sowohl solche Krankheitsbilder umfasst, deren Ursache in einer Organdisfunktion oder -krankheit liegt als auch solche, die auf Krankheiten oder Störungen des zentralen Nervensystems zurückzuführen sind. Demgemäß wird jede Behandlung von Blasenfunktionsstörung, insbesondere Harninkontinenz jeglicher Art durch die vorliegende Erfindung in Betracht gezogen.

Damit umfasst eine weitere Ausführungsform der vorliegenden Erfindung die Verwendung der erfindungsgemäßen Zusammensetzung zur Herstellung eines Medikaments zur Behandlung oder Verhütung einer jeden, der im vorstehenden Paragraphen genannten Indikationen zu Blasenfehlfunktionen.

Die Behandlung der obigen Krankheiten oder Störungen wird durch Zufuhr einer therapeutisch wirksamen Menge der erfindungsgemäßen Zusammensetzung an einen Säuger bewerkstelligt. In den meisten Fällen ist dies ein Mensch, aber die Behandlung von Nahrungstieren (z.B. Vieh) und Haustieren (z.B. Hunden, Katzen und Pferden) wird ausdrücklich hierin abgedeckt. Für die verterinärmedizinische Verwendungen können die zu verwendenden Dosierungen andere sein, als die hierin angegebenen Dosierungen.

Es wird erwartet, dass die neue Zusammensetzung mit einem minimalen Grad an schädlichen Nebenwirkungen bei denjenigen für eine rasche Erleichterung sorgen, die an den obigen Krankheiten und Störungen leiden.

### e) Beispiele

Die Erfindung wird durch die folgenden Beispiele anschaulicher beschrieben.

Duloxetin und (-)-Ethyl-2-[4-(2-{[(1S,2R)-2-hydroxy-2-(4-hydroxyphenyl)-1-methyl-ethyl]amino} ethyl)-2,5-dimethylphenyloxy]acetat.

Duloxetin und (-)-Ethyl-2-[4-(2-{[(1S,2R)-2-hydroxy-2-(4-hydroxyphenyl)-1-methyl-ethyl]amino}ethyl)-2,5-dimethylphenyloxy]acetat-monohydrochlorid.

Duloxetin und (-)-2-[4-(2-{[(1S,2R)-2-Hydroxy-2-(4-hydroxyphenyl)-1-methylethyl]-amino} ethyl)-2,5-dimethylphenyloxy]essigsäure.

Duloxetin und (-)-2-[4-(2-{[(1S,2R)-2-Hydroxy-2-(4-hydroxyphenyl)-1-methylethyl]-amino}ethyl)-2,5-dimethylphenyloxy]essigsäure-monohydrochlorid.
Nachdem die Erfindung in Einzelheiten und mit Bezug auf die bevorzugten Ausführungsformen derselben beschrieben worden ist, ist es offensichtlich, dass Modifikationen und Abwandlungen möglich sind, ohne vom Bereich der beigefügten Ansprüche abzuweichen.

**Beispiel N°1** Zusammensetzung aus (-)-Ethyl-2-[4-(2-{[(1S,2R)-2-hydroxy-2-(4-hydroxyphenyl)-1-methylethyl] amino} ethyl)-2,5-dimethylphenyloxy]acetat-monohydrochlorid und Duloxetin: Filmtablette 20 mg / 20 mg

**Kern**

| Bestandteile | **mg/Tablette** |
|---|---|
| (-)-Ethyl-2-[4-(2-{[(1S,2R)-2-hydroxy-2-(4-hydroxyphenyl)-1-methylethyl]amino ethyl)-2,5-dimethylphenyloxy] acetat-monohydrochlorid | 21.820 |
| Duloxetin Hydrochlorid | 22.460 |
| Lactose-monohydrat | 78.120 |
| Avicel (PH 101) | 45.000 |
| Maisstärke | 6.300 |
| Gereinigtes Wasser | (q.s.) |
| Natriumstärkeglykolat | 3.600 |
| Magnesiumstearat | 1.800 |
| Hochdisperses Siliciumdioxid | 0.900 |

**Trennschicht**

| Bestandteile | **mg/Tablette** |
|---|---|
| Hydroxypropylmethylcellulose 2910 | 2.750 |
| Polyethylenglykol 400 | 0.325 |
| Titandioxid | 1.000 |
| Talcum | 0.925 |
| Gereinigtes Wasser | (q.s.) |

**Magensaftresistenter Überzug**

| Bestandteile | **mg/Tablette** |
|---|---|
| Sureteric (Polyvinylacetatphthalat) | 14.950 |
| Siliconemulsion | 0.050 |
| Gereinigtes Wasser | (q.s.) |
| | |
| **Gesamtgewicht der Tablette** | **200.000** |

### Beispiel N°2 Zusammensetzung aus (-)-Ethyl-2-[4-(2-{ [(1S,2R)-2-hydroxy-2-(4-hydroxyphenyl)-1-methylethyl]amino} ethyl)-2,5-dimethylphenyloxy]acetatmonohydrochlorid und Duloxetin: Filmtablette 80 mg / 60 mg

**Kern**

| Bestandteile | **mg/Tablette** |
|---|---|
| (-)-Ethyl-2-[4-(2-{[(1S,2R)-2-hydroxy-2-(4-hydroxyphenyl)-1-methylethyl]amino} ethyl)-2,5-dimethylphenyloxy] acetat-monohydrochlorid | 87.280 |
| Duloxetin Hydrochlorid | 67.380 |
| Lactose-monohydrat | 185.340 |
| Avicel (PH 101) | 125.000 |
| Hydroxypropylmethylcellulose | 10.000 |
| Gereinigtes Wasser | (q.s.) |
| Crospovidone | 10.000 |
| Magnesiumstearat | 10.000 |
| Hochdisperses Siliciumdioxid | 5.000 |

**Magensaftresistenter Überzug**

| Bestandteile | **mg/Tablette** |
|---|---|
| Eudragit L 30 D-55 (Copolymerisat aus Methacrylsäure und Ethylacrylat) | 136.000+ |
| Triethylcitrat | 4.000 |
| Polysorbat 80 | 4.000 |
| Glycerinmonostearat | 1.200 |
| Gereinigtes Wasser | (q.s.) |
| **Gesamtgewicht der Tablette** | **550.000** |

| | |
|---|---|
| ⁺ Eudragit L 30 D-55 ist eine 30%ige Suspension. Der Wasseranteil wird während des Aufsprühens entfernt. | |

**Beispiel N°3** Zusammensetzung von (-)-Ethyl-2-[4-(2-{[(1S,2R)-2-hydroxy-2-(4-hydroxyphenyl)-1-methylethyl]amino}ethyl)-2,5-dimethylphenyloxy] acetatmonohydrochlorid und Duloxetin: Hartgelatinekapsel gefüllt mit Pellets 20 mg / 20 mg

**Duloxetin Pellets**

| Bestandteile | **mg/Tablette** |
|---|---|
| Duloxetin Hydrochlorid | 22.460 |
| Mikrokristalline Cellulose Pellets | 60.000 |
| β-Lactose | 80.040 |
| Hydroxypropylcellulose | 1.500 |
| Crospovidone | 6.000 |
| Gereinigtes Wasser | (q.s.) |

**Trennschicht**

| Bestandteile | **mg/Tablette** |
|---|---|
| Hydroxypropylmethylcellulose | 3.400 |
| Gereinigtes Wasser | (q.s.) |

**Magensaftresistenter Überzug**

| Bestandteile | **mg/Tablette** |
|---|---|
| HPMCAS-LF | 20.400 |
| Triethylcitrat | 4.080 |
| Talkum | 2.120 |
| Gereinigtes Wasser | (q.s.)* |

**(-)-Ethyl-2-[4-(2-{[(1S,2R)-2-hydroxy-2-(4-hydroxyphenyl)-1-methyl-ethyl]amino}ethyl)-2,5-dimethylphenyloxy]acetat-monohydrochlorid Pellets**

| Bestandteile | **mg/Tablette** |
|---|---|
| (-)-Ethyl-2-[4-(2-{[(1S,2R)-2-hydroxy-2-(4-hydroxyphenyl)-1-methylethyl]amino}ethyl)-2,5-dimethylphenyloxy]acetat-monohydrochlorid | 21.820 |
| Mikrokristalline Cellulose Pellets | 60.000 |
| Hydroxypropylcellulose | 0.800 |
| Talkum | 2.380 |
| Gereinigtes Wasser . | (q.s.) |

**Filmüberzug**

| Bestandteile | **mg/Tablette** |
|---|---|
| Hydroxypropylmethylcellulose | 4.250 |
| Gereinigtes Wasser | (q.s.) |

**Kapsel**

| Bestandteile | **mg/Tablette** |
|---|---|
| Duloxetin Pellets mit magensaftresist. Überzug | 200.000 |
| (-)-Ethyl-2-[4-(2-{[(1S,2R)-2-hydroxy-2-(4-hydroxyphenyl)-1-methylethyl]amino }ethyl)-2,5-dimethylphenyloxy]acetat-monohydrochlorid Pellets mit Filmüberzug | 89.250 |
| Hartgelatinekapsel (Größe 0) | 98.000 |
| | |
| **Gesamtgewicht der Kapsel** | **387.250** |

## Patentansprüche

1. Zusammensetzung umfassend (a) eine pharmazeutisch wirksame Menge Duloxetin, das entweder in seiner racemischen oder (+)-enantiomeren Form vorliegt und (b) (-)-Ethyl-2-[4-(2-{[(1S,2R)-2-hydroxy-2-(4-hydroxyphenyl)-1-methylethyl]amino}ethyl)-2,5-dimethylghenyloxy]acetat und/oder (-)-2-[4-(2-{[(1S,2R)-2-Hydroxy-2-(4-hydroxyphenyl)-1-methylethyl]amino}ethyl)-2,5-dimethylphenyloxy]essigsäure und/oder ein pharmazeutisch annehmbares Salz derselben.

2. Zusammensetzung nach Anspruch 1, die 0,1 mg bis 500 mg Duloxetin und 10 mg bis 750 mg Komponente (b) enthält.

3. Zusammensetzung nach irgendeinem der Ansprüche 1 bis 2, in der die Komponente (a) und die Komponente (b) in derselben Applikationsform formuliert sind.

4. Zusammensetzung nach irgendeinem der Ansprüche 1 oder 2, in der die Komponente (a) und die Komponente (b) in verschiedenen Applikationsformen formuliert sind.

5. Zusammensetzung nach einem der Ansprüche 1 bis 4 als Medikament

6. Zusammensetzung nach Anspruch 5 geeignet zur rektalen, vaginalen, topischen, oralen, sublingualen, intranasalen, transdermalen oder parenteralen Applikation.

7. Zusammensetzung nach Anspruch 5 oder 6 geeignet zur simultanen Verabreichungen der beiden Komponenten (a) und (b).

8. Zusammensetzung nach Anspruch 5 oder 6, wobei wenigstens einer der beiden Komponenten wenigstens teilweise verzögert frei gesetzt wird.

9. Zusammensetzung nach Anspruch 7 oder 8, wobei wenigstens einer der beiden Komponenten wenigstens teilweise sofort frei gesetzt wird.

10. Verwendung einer Zusammensetzung nach einem der Ansprüche 1 bis 9 zur Herstellung eines Medikaments zur Behandlung von Blasenfunktionsstörungen ausgewählt ist aus der Gruppe bestehend aus Harninkontinenz, Dranginkontinenz, Stressinkontinenz, Mischinkontinenz, anderen Formen von Harninkontinenz und/oder hyperaktiver Blase.

11. Verwendung einer Zusammensetzung enthaltend die Komponente (a) nach Anspruch 1, die nicht die Komponente (b) enthält in Kombination mit einer zweiten Zusammensetzung enthaltend die Komponente (b) nach Anspruch 1, die nicht die Komponente (a) enthält zur Herstellung eines Medikaments zur Benhandlung von Blasenfünktionsstörungen nach Anspruch 10.

## Claims

1. Composition comprising: (a) a pharmaceutically effective amount of duloxetine which is present in either its racemic or (+)-enantiomeric form and (b) (-)-ethyl-2-[4-(2-{[(1S,2R)-2-hydroxy-2-(4-hydroxyphenyl)-1-methylethyl]-amino}-ethyl)-2,5-dimethylphenyloxy] acetate and/or (-)-2-[4-(2-{[(1S,2R)-2-hydroxy-2-(4-hydroxyphenyl)-1-methylethyl]amino}ethyl)-2,5-dimethylphenyloxy] acetic acid and/or a pharmaceutically acceptable salt thereof.

2. Composition according to claim 1 which contains 0.1 mg to 500 mg of duloxetine and 10 mg to 750 mg of component (b).

3. Composition according to any one of claims 1 or 2, wherein component (a) and component (b) are formulated in the same preparation.

4. Composition according to any one of claims 1 to 3, wherein component (a) and component (b) are formulated in different preparations.

5. Composition according to one of claims 1 or 2 as a medicament.

6. Composition according to claim 5 suitable for rectal, vaginal, topical, oral, sublingual, intranasal, transdermal or parenteral administration.

7. Composition according to claim 5 or 6 suitable for administering the two components (a) and (b) simultaneously.

8. Composition according to claim 5 or 6, wherein at least one of the two components is at least partially released after some delay.

9. Composition according to claim 7 or 8, wherein at least one of the two components is at least partially released immediately.

10. Use of a composition according to one of claims 1 to 9 for preparing a medicament for treating bladder function disorders selected from the group consisting of urinary incontinence, urge incontinence, stress incontinence, mixed incontinence, other forms of urinary incontinence and/or hyperactive bladder.

11. Use of a composition containing component (a) according to claim 1 which does not contain component (b) in conjunction with a second composition containing component (b) according to claim 1 which does not contain component (a), for preparing a medicament for treating bladder function disorders according to claim 10.

## Revendications

1. Composition comprenant (a) une quantité pharmaceutiquement efficace de duloxétine, qui est sous sa forme racémique ou d'énantiomère (+) et (b) du (-)-2-[4-(2-{[(1S,2R)-2-hydroxy-2-(4-hydroxyphényl)-1-méthyléthyl]-amino}éthyl)-2,5-diméthylphényloxy]acétate d'éthyle et/ou de l'acide (-)-2-[4-(2-{[(1S,2R)-2-hydroxy-2-(4-hydroxyphényl)-1-méthyléthyl]amino}éthyl)-2,5-diméthylphényloxy]acétique et/ou un sel pharmaceutiquement acceptable de celui-ci.

2. Composition selon la revendication 1 qui contient 0,1 mg à 500 mg de duloxétine et 10 mg à 750 mg de composant (b).

3. Composition selon l'une quelconque des revendications 1 à 2 où le composant (a) et le composant (b) sont formulés dans la même forme d'application.

4. Composition selon l'une quelconque des revendications 1 ou 2 où le composant (a) et le composant (b) sont formulés dans des formes d'application différentes.

5. Composition selon l'une des revendications 1 à 4 comme médicament.

6. Composition selon la revendication 5 appropriée à l'application rectale, vaginale, topique, orale, sublinguale, intranasale, transdermique ou parentérale.

7. Composition selon la revendication 5 ou 6 appropriée à l'administration simultanée des deux composants (a) et (b).

8. Composition selon la revendication 5 ou 6 où au moins l'un des deux composants est libéré au moins en partie de manière retardée.

9. Composition selon la revendication 7 ou 8 où au moins l'un des deux composants est libéré au moins partiellement immédiatement.

10. Utilisation d'une composition selon l'une des revendications 1 à 9 pour la fabrication d'un médicament pour le traitement des troubles de la fonction vésicale, choisis dans le groupe consistant en l'incontinence urinaire, l'incontinence impérieuse, l'incontinence d'effort, l'incontinence mixte, d'autres formes d'incontinence urinaire et/ou de vessie hyperactive.

11. Utilisation d'une composition contenant le composant (a) selon la revendication 1, qui ne contient pas le composant (b) en combinaison avec une seconde composition contenant le composant (b) selon la revendication 1, qui ne contient pas le composant (a) pour la fabrication d'un médicament pour le traitement des troubles de la fonction vésicale selon la revendication 10.
